# FASCICULE DE BREVET EUROPEEN

(11) **EP 3 613 728 B1**
(45) Date de publication et mention de la délivrance du brevet: **09.12.2020**
(21) Numéro de dépôt: 19203533.5
(22) Date de dépôt: 18.07.2014
(51) Int. Cl.: C07C 235/10, C07C 235/14, C07C 235/16, C09D 4/00

(54) **DIAMIDES D'ACIDES GRAS A BASE DE DIAMINES CYCLOALIPHATIQUES ET ALIPHATIQUES, UTILISES COMME ORGANOGELATEURS**
FETTSÄUREDIAMIDE AUF DER BASIS VON ZYKLOALIPHATISCHEN UND ALIPHATISCHEN DIAMINEN, DIE ALS ORGANOGELBILDNER VERWENDET WERDEN
FATTY ACID DIAMIDES MADE OF CYCLOALIPHATIC AND ALIPHATIC DIAMINES, USED AS ORGANOGELATORS

(30) Priorité: 25.07.2013 FR 1357347
(43) Date de publication de la demande: 26.02.2020
(62) Demande divisionnaire de: 14790148.2
(73) Titulaire: ARKEMA FRANCE, 92700 Colombes (FR)
(72) Inventeur: BERNARD, Michael Y., 60550 Verneuil en Halatte (FR)
(74) Mandataire: Arkema Patent

(56) Documents cités:
- EP-A1- 2 098 502
- JP-A- 10- 328 882
- US-A- 3 977 894

## Description

L'invention concerne d'abord un diamide d'acide gras comprenant dans sa structure à la fois des diamines cycloaliphatiques et aliphatiques avec un ratio molaire spécifique et l'utilisation de ce produit comme organogélateur ou comme agent ou additif de rhéologie, en particulier dans des compositions de revêtements, de moulage, de mastics ou d'agents d'étanchéité, d'agent décapant ou de cosmétique.

US 3 977 894 décrit des additifs de rhéologie auto-activables à base de mélange homogène d'organoargiles, de glycéryl tri-12-hydroxystéarate et d'un amide gras à base d'une diamine aliphatique en C₂ à C₁₈ et d'acide 12-hydroxystéarique.

Des additifs de rhéologie, en particulier de thixotropie à base de diamides comprenant comme composant diamine, une amine aliphatique et la xylylène diamine et en particulier la m-xylylène diamine, sont déjà connus avec des performances intéressantes. Cependant, compte tenu des contraintes environnementales liées à sa toxicité et surtout ses effets potentiels sur la santé lors de sa manipulation, la question de sa substitution au moins partielle à taux plus réduit et de préférence de sa substitution totale se pose. Une telle substitution ne doit cependant pas affecter de manière significative les performances rhéologiques des diamides gras obtenus. Plus particulièrement, les diamides visés doivent avoir des performances élevées, en termes d'effet thixotrope et de résistance à la coulure, avec des conditions d'activation imposées à relativement basse température, c'est-à-dire de 40°C à moins de 80°C. Ceci correspond en particulier au cas des compositions de liant en milieu solvant organique réactif ou non réactif, lequel solvant ne doit pas par effet d'évaporation ou par effet de réaction subir des températures d'activation élevées et ne doit pas présenter de risque pour l'environnement et la santé s'il y a émission de vapeurs de solvant à température élevée ni de risque pour la stabilité de la composition de liant si ledit solvant est susceptible de réagir par chauffage à température plus élevée. En plus, aucune affectation significative des performances finales d'application ne doit avoir lieu. Ce problème est surtout valable pour les compositions de revêtements comme les peintures, vernis, encres et enduits gélifiés (couramment appelés aussi « gel coats ») ou de colles ou d'adhésifs ou d'agent décapant ou de moulage ou de cosmétique en milieu solvant organique réactif ou non réactif. Par exemple, un solvant type non réactif, utilisé dans les peintures est le xylène. Comme exemple de solvant réactif (appelé aussi diluant réactif), on peut citer le styrène ou des monomères acryliques dans des compositions d'enduits gélifiés (gel coats) ou de moulage à base de polyesters insaturés ou des vinyl esters.

L'affinité de la m-xylylène diamine et des diamides à base de cette diamine et d'acide 12-hydroxy stéarique par rapport aux solvants utilisés dans les compositions d'application, en particulier dans les revêtements ou colles ou adhésifs, fait que cette diamine est un composant essentiel pour des additifs organogélateurs ou des additifs de rhéologie, convenables pour ces applications. Plus particulièrement, dans les revêtements comme les peintures, vernis et encres en milieu solvant non réactif, comme le xylène, la question de son remplacement demande à trouver un bon compromis de performances dans ce solvant et avec les liants organiques utilisables dans ce milieu, ceci sans affecter de manière significative les performances rhéologiques lors de l'application finale.

En fonction des paramètres liés au milieu d'utilisation, des paramètres liés à la composition spécifique (paramètres moléculaires et de structure) et d'autres paramètres liés aux conditions d'activation dans un système donné, les performances rhéologiques peuvent varier significativement et il est très difficile d'extrapoler et de prédire ces performances d'une manière générale à partir de ce qui est connu pour les performances d'un système connu très particulier. L'effet de gélation dans un milieu particulier d'application visée nécessite un compromis spécifique entre l'aptitude de l'amide à se solubiliser (avec une solubilité visée spécifiquement limitée) et liée à l'aptitude à former des fibres cristallines et la tendance à la précipitation et sédimentation dans ledit milieu à base dudit solvant organique. Le terme solvant dans les définitions données ci-dessus et par la suite est à comprendre comme un solvant organique principal qui ne peut pas subir (n'est pas apte à) une activation à haute température, en particulier supérieure à 90°C. Par conséquent, ledit solvant selon l'invention est apte à être activé qu'à des températures inférieures à 90°C.

Ainsi, la présente invention a comme but de surmonter les inconvénients exposés et vise donc à mettre au point des nouveaux diamides gras de composition spécifique et sélective. Cette composition comprend des diamines de structure cycloaliphatique en remplacement partiel et plus particulièrement en remplacement total de xylylène diamines, en particulier de la m-xylylène diamine en présence d'au moins une diamine aliphatique comme composants essentiels amines desdits diamides dans des rapports spécifiques. L'invention vise ainsi des performances d'agent organogélateur satisfaisantes et en particulier au moins aussi bonnes que celles des organogélateurs à base de xylylène diamine seule (100% du composant amine), c'est-à-dire sans aucun remplacement par ladite diamine cyclique, pris comme référence de comparaison. Les diamides selon l'invention sont particulièrement aptes à être activés à basse température comme décrit ci-haut.

Les avantages de la présente invention, par rapport aux produits et utilisations de l'état de la technique dans le domaine des compositions de liant en milieu solvant organique, sont des performances rhéologiques satisfaisantes et de préférence au moins aussi bonnes que celles obtenues avec les amides à base de m-xylylène diamine seule (en absence totale de diamine cycloaliphatique) avec des performances au moins équivalentes. Ces performances sont obtenues sans avoir les contraintes environnementales et de santé liées à la toxicité de ladite diamine aromatique.

La présente invention couvre d'abord ledit diamide de composition spécifique.

Le deuxième objet de l'invention concerne l'utilisation dudit diamide comme organogélateur.

Un autre objet est l'organogélateur comprenant ledit diamide.

Finalement, l'invention concerne une composition de liant comprenant ledit diamide pour diverses utilisations.

Donc, le premier objet de l'invention concerne un diamide d'acide gras caractérisé qui comprend au moins un produit de réaction obtenu à partir d'un mélange réactionnel comprenant :
a) au moins une diamine cycloaliphatique comprenant un cycle en 6 atomes de carbone et en particulier comprenant globalement, y compris ledit cycle, de 6 à 18 atomes de carbone, de préférence ladite diamine étant choisie parmi : la bis(aminométhyl)-1,3 cyclohexane (1,3-BAC), la bis(aminométhyl)-1,4 cyclohexane (1,4-BAC), la bis(aminométhyl)-1,2 cyclohexane (1,2-BAC) et leurs mélanges d'isomères, décahydronaphtalène diamines, isophorone diamines, plus préférentiellement la bis(aminométhyl)-1,3 cyclohexane (1,3-BAC), la bis(aminométhyl)-1,4 cyclohexane (1,4-BAC), et encore plus préférentiellement la la bis(aminométhyl)-1,3 cyclohexane (1,3-BAC),
b) un hydroxy acide gras parmi l'acide 12-hydroxy stéarique (12-HSA), l'acide 9-hydroxy stéarique (9-HSA), l'acide 10-hydroxystéarique (10-HSA), l'acide 14-hydroxy eicosanéique (14-HEA) ou leurs mélanges binaires ou ternaires ou quaternaires, de préférence l'acide 12-hydroxy stéarique ou un mélange binaire ou ternaire de l'acide 12-hydroxy stéarique avec les autres hydroxyacides cités,
c) au moins une deuxième diamine sélectionnée parmi les diamines primaires aliphatiques linéaires en C₂ à C₁₂, de préférence en C₂ à C₈, plus préférentiellement en C₂ à C₆,
d) en option, au moins un monoacide sélectionné parmi les acides carboxyliques linéaires saturés et non hydroxylés en C₆ à C₁₈, de préférence de C₆ à C₁₅, plus préférentiellement de C₆ à C₁₂,
e) en option, au moins une troisième diamine primaire différente de c) sélectionnée parmi les diamines aliphatiques linéaires en C₂ à C₁₂, de préférence en C₂ à C₈, plus préférentiellement en C₂ à C₆,
f) au moins une diamine aromatique choisie parmi les xylylène diamines, de préférence parmi m-, p-xylylène diamines (m-, p-XDA), plus préférentiellement la m-xylylène diamine (m-XDA), ladite diamine aromatique pouvant être remplacée partiellement ou totalement par une diamine cycloaliphatique telle que définie selon a).

Selon une option préférée, le % molaire de a/(a+f) est d'au moins 50% et va jusqu'à 100% et le pourcentage molaire de (a+f) / (a+c+e+f) qui représente le rapport molaire des amine cycloaliphatique a) et aromatique f) par rapport à l'ensemble des amines, varie de 30 à 80% et de préférence de 30 à 65%. Plus particulièrement, le rapport molaire a/(c+e) de la diamine a) sur les autres diamines aliphatiques est d'au moins 0,25 et de préférence varie de 0,45 à 0,95, plus préférentiellement de 0,5 à 0,85.

Le terme « linéaire » n'a de signification par rapport à une structure ramifiée, que pour les chaînes comprenant au moins 4 atomes de carbone et signifie pour ces chaînes l'absence de toute ramification, les chaînes de longueur inférieure (C₂ et C₃) étant par définition toujours linéaires.

Selon une autre possibilité de la présente invention, ladite diamine cycloaliphatique a), de préférence la 1,3-BAC ou 1,4-BAC, est en présence de xylylène diamines f), de préférence p-XDA ou m-XDA, plus préférentiellement m-XDA, avec ladite diamine a) représentant de 50 à 99% en moles du total en moles de diamine a)+xylylène diamines f), de préférence de 60 à 99% dudit total.

Selon une option alternative et plus préférée de l'invention, ladite diamine f) est absente (0%) avec un rapport molaire a/(a+f) de 100%, de préférence avec ladite diamine cycloaliphatique a) étant la 1,4-BAC ou la 1,3-BAC. Ladite diamine a) peut être présente seule ou sous forme de mélange comprenant ladite diamine a).

Selon une option plus particulière, ledit diamide selon l'invention comprend :
i) un diamide consistant du produit de réaction de 1 mole de ladite diamine a) avec 2 moles dudit hydroxy acide b) comme défini ci-haut, de préférence ledit hydroxy acide b) étant l'acide hydroxy-12 stéarique et
ii) un diamide consistant du produit de réaction de 1 mole de diamine c) avec 2 moles dudit hydroxy acide b) comme défini ci-haut, de préférence ledit hydroxy acide b) étant l"acide hydroxy-12 stéarique et
iii) un diamide consistant du produit de réaction de 1 mole de diamine selon e) et de 2 moles dudit hydroxy acide b) comme défini ci-haut, de préférence ledit hydroxy acide b) étant l'acide hydroxy-12 stéarique.

Le diamide de l'invention peut comprendre au moins 2, de préférence au moins 3, produits de réaction différents tels qu'issus de la réaction entre lesdites diamines a), c), e) et éventuellement des xylylène diamines f) et les monoacides b) et éventuellement d).

Selon un mode particulier, ledit monoacide d) est présent à un taux tel que le rapport molaire d/(b+d) est inférieur à 0,5 et de préférence il varie entre 0,02 et 0,5.

Selon une préférence particulière, ledit diamide de l'invention est sous forme de poudre micronisée, de préférence sous forme de poudre ayant une taille moyenne en volume inférieure à 20 µ, plus préférentiellement inférieure à 15 µ. La détermination de ladite taille peut être réalisée avec un appareil de mesure équipé d'un détecteur laser.

La micronisation est réalisée par broyage mécanique suivie éventuellement d'un tamisage ou par broyage à jet d'air pour l'obtention des poudres les plus fines avec une distribution de granulométrie contrôlée et plus étroite.

Le second objet de l'invention concerne l'utilisation du diamide de l'invention en tant qu'agent organogélateur en milieu solvant organique.

Ainsi, ledit additif peut être utilisé en tant qu'additif de rhéologie, en particulier sous forme de pâte préactivée préconcentrée dans un solvant organique. Le solvant organique peut être un mélange de solvants. De préférence, il est un solvant polaire ou comprend au moins un solvant polaire. Une utilisation importante préférée dudit diamide est une utilisation (comme additif de rhéologie) dans une composition de revêtements en particulier dans les peintures, vernis, encres et enduits gélifiés (« gel coats ») ou dans une composition de colle et d'adhésif ou d'agent décapant ou de moulage ou de cosmétique. Plus préférentiellement, ladite utilisation concerne une composition de revêtements, compte tenu des questions posées d'affinité dans ces milieux de la diamine m-xylylène diamine à remplacer selon l'objectif de l'invention, comme expliqué ci-haut.

Un autre objet de l'invention est un agent organogélateur, en particulier additif de rhéologie qui comprend au moins un diamide tel que défini selon l'invention, comme décrit ci-dessus. Plus particulièrement, il peut s'agir d'un agent ou d'un additif de rhéologie en particulier thixotrope dans une composition préconcentrée dans au moins un solvant organique, y compris un mélange de solvants, de préférence dans au moins un solvant organique polaire, sous forme de pâte préactivée.

Le terme « solvant organique polaire » selon la description ci-haut inclut dans sa définition ou est à interpréter comme signifiant « au moins un solvant organique polaire » ou « un mélange de solvants organiques comprenant au moins un solvant organique polaire ». Est considéré comme solvant organique polaire, un solvant comprenant au moins un groupement polaire, comme par exemple un groupement alcool ou ester. Comme exemple de solvant organique polaire selon la première option d'interprétation, on peut citer un alcool tel que l'éthanol ou le butanol ou leur mélange et selon la deuxième option d'interprétation (mélange de solvants organiques comprenant au moins un solvant organique polaire), un mélange d'un tel alcool (éthanol ou butanol) avec un solvant non polaire comme par exemple le xylène.

Finalement, l'invention couvre une composition de liant organique, laquelle comprend en tant qu'agent (ou additif étant un terme équivalent à agent) de rhéologie au moins un diamide comme défini ci-haut selon l'invention.

Plus particulièrement, cette composition de liant organique est une composition de revêtements, de peintures, de vernis, d'encres et d'enduits gélifiés (« gel coats ») ou une composition de colle ou d'adhésif ou d'agent décapant ou de moulage ou de cosmétique. Selon une option plus préférée, ladite composition est une composition de revêtements. Selon une autre possibilité, ladite composition est une composition de mastic ou d'agent d'étanchéité. Selon une autre option, cette composition est une composition de moulage. Les compositions de moulage ou d'enduits gélifiés (« gel coats ») ou de revêtements réticulables par rayonnement sont des compositions en milieu solvant réactif (appelé également diluant réactif), comme les polyesters insaturés ou vinyl esters dans le styrène ou dans un autre comonomère équivalent ou des monomères et/ou oligomères acryliques (signifiant acrylés) multifonctionnels, lesdits oligomères utilisant comme diluant réactif un monomère acrylique multifonctionnel.

Plus spécifiquement, ledit liant organique peut être sélectionné parmi : résines époxy, polyesters insaturés, vinyl esters, alkydes, résines silanées, polyuréthanes, polyesters-amides, résines acryliques solvantées (diluant non réactif), monomères et/ou oligomères acryliques (multifonctionnels) avec dans le dernier cas le diluant réactif étant un monomère acrylique (multifonctionnel) ou élastomères chlorés et élastomères non chlorés et polymères chlorés autres qu'élastomères chlorés, de préférence, résines époxy, polyesters insaturés, vinyl esters, alkydes, polyuréthanes, polyesters-amides, résines acryliques solvantées et lesdits monomères et/ou oligomères acryliques ou élastomères chlorés et élastomères non chlorés.

Monomères et/ou oligomères acryliques signifient porteurs d'au moins une fonction acrylate ou méthacrylate et en particulier multifonctionnels, c'est-à-dire avec au moins deux desdites fonctions acrylate ou méthacrylate par molécule.

Les exemples présentés ci-dessous sont à titre d'illustration de l'invention et de ses performances et ne limitent en rien sa couverture.

### Partie expérimentale

### I - Matières premières utilisées

**Tableau 1 : Matières premières utilisées**

| **Produit** | **Fonction** | **Référence commerciale** | **Fournisseur** |
|---|---|---|---|
| Hexaméthylènediamine | Composant vs amide | Hexaméthylènediamine 98% | Aldrich |
| Meta -xylylènediamine | Composant vs amide | mXDA | Mitsubishi Chemicals |
| 1,3-bis(aminométhyl) cyclohexane | Composant vs amide | 1,3-BAC | Mitsubishi Chemicals |
| Acide 12 hydroxy stéarique | Composant vs amide | 12-HSA | Jayant Agro |
| Ethylène diamine | Composant vs amide | Ethylène diamine ≥ 99,5% (GC) | Aldrich |
| Résine époxy | Liant vs formulation | Araldite® GZ 7071 X75 | Huntsman |
| Résine époxy | Liant vs formulation | Araldite® GY 783 BD | Huntsman |
| Agent débullant | Agent débullant | BYK® A530 | Byk |
| Agent Dispersant | Dispersant | Disperbyk® 110 | Byk |
| Dioxide de titane | Pigment | Tiona® 595 | Société des ocres de France |
| Oxyde de fer | Pigment | Bayferrox® 915 | Lubrizol |
| Phosphate de zinc | Pigment | ZP 10 | HEUCOPHOS |
| Talc | Additif vs formulation | Finntalc® MO5 | Mondo minerais |
| Silice | charge | HPF6 | Sibelco |
| n-Butanol | Solvant | n-Butanol | Aldrich |
| Polyamide | Durcisseur vs liant | CRAYAMID® 140 | Arkema |
| Xylène | solvant | Xylène, reagent grade | Aldrich |

### II - Méthodes et tests utilisés

Les formulations sont évaluées avec deux tests : le test de résistance à l'écoulement (coulure) et une évaluation de la viscosité à différentes vitesses.

### - Test de résistance à l'écoulement

Il s'effectue à l'aide d'un contrôleur de coulure (Levelling/Sagging Tester de Sheen Instruments) qui permet d'établir la résistance d'un revêtement à la coulure due à la gravité. Fabriqué en inox et doté d'une lame droite, ce contrôleur comporte des encoches de valeur croissante.

Le test consiste à déposer différentes bandes de peinture d'épaisseur parallèle sur une bande de contraste grâce au contrôleur de coulure. La carte de contraste est immédiatement placée en position verticale, le film le plus fin en haut. L'épaisseur à laquelle les bandes se rejoignent indique la tendance à la coulure.

### - Evaluation de la viscosité

Elle s'effectue ici à l'aide d'un Brookfield® RV à 25°C (mobile : S 4). La vitesse du mobile est fixée à 50 RPM (rotations par minute) et l'on mesure la viscosité de chaque peinture après que celle-ci est stabilisée. On répète l'opération pour une vitesse de 20 RPM, 10 RPM, 5 RPM, 1 RPM.

### III - Préparation et caractérisation des diamides (ou organogélateurs, additifs de rhéologie)

### EXEMPLE 1 : Amide à base de méta-xylylène diamine et d'hexaméthylène diamine et de 12-HSA

Dans un ballon de 1-Litre équipé d'un thermomètre, un Dean-Stark, un condensateur et d'un agitateur, on introduit sous un courant d'azote, 61,29 g de méta-xylylène diamine (0,46 moles), 63,8 g de éthylène diamine (soit 0,54 moles), 315,2 g d'acide 12-hydroxy stéarique (1,00 mole).

Le mélange est chauffé à 200°C toujours sous courant d'azote. L'eau éliminée commence à s'accumuler dans le Dean Stark dès 150°C. La réaction est contrôlée par l'indice d'acide et d'amine. Lorsque les valeurs d'acides et d'amines sont inférieures à 10 (en mg KOH/g), le mélange réactionnel est refroidi à 150°C, puis déchargé dans un moule siliconé. Une fois refroidi à la température ambiante, le produit est micronisé mécaniquement par broyage et tamisé pour obtenir une granulométrie fine et contrôlée avec taille moyenne obtenue de 7 µm.

Pour les EXEMPLES 2, 3 et 4 : le même mode opératoire a été utilisé mais avec les proportions de composants de réaction présentés dans le tableau 2 ci-dessous :

**Tableau 2 : produits amides préparés 1 à 4**

| **Exemple** | **Réactifs amines/ acides** | **Mole** |
|---|---|---|
| 1 | Méta-xylylène diamine | 0,46 |
| | Hexaméthylène diamine | 0,54 |
| | Acide 12-hydroxystéarique | 1,00 |
| 2 | Méta-xylylène diamine | 0,42 |
| | Ethylène diamine | 0,18 |
| | Hexaméthylène diamine | 0,40 |
| | Acide 12-hydroxystéarique | 1,00 |
| 3 | 1,3- bis (aminométhyl) cyclohexane | 0,46 |
| | Hexaméthylène diamine | 0,54 |
| | Acide 12-hydroxystéarique | 1,00 |
| 4 | 1,3- bis (aminométhyl) cyclohexane | 0,42 |
| | Ethylène diamine | 0,18 |
| | Hexaméthylène diamine | 0,40 |
| | Acide 12-hydroxystéarique | 1,00 |

### IV - Evaluation des performances rhéologiques dans une formulation de peinture

Les amides préparés ont été évalués dans des formulations (réactives) de peintures époxy à haut taux de solides (ou haut extrait sec) dans le xylène.

### 1) Préparation des formulations de peinture

Une formulation dite « millbase » est préparée avec les proportions présentées au tableau 3 ci-dessous et de la manière suivante :
Dans un bol de disperseur (Dispermill® 2075 yellow line, fournisseur : Erichsen) chauffé par un système de double enveloppe, on effectue les opérations suivantes et successives :
1.1) Introduction des liants époxy ainsi que du dispersant et de l'agent débullant. L'homogénéisation se fait pendant 2 minutes à 800 tours/minute (800 rotations par minute ou 800 rpm).
1.2) Introduction des charges et des pigments, puis broyage à 3000 rpm pendant 30 minutes à l'aide d'une pâle de 7 cm. Le bol à double enveloppe permet que cette étape ait lieu à température ambiante avec un bain d'eau froide (à 20°C).
1.3) Introduction des solvants (butanol selon tableau 3) et homogénéisation

### 2) Activation de l'additif amide dans la « millbase »

24 heures après la préparation de la millbase, la formulation est de nouveau dispersée à l'aide d'une pâle de 4 cm à 3000 rpm. Le diamide à évaluer est introduit dans la millbase et activé in situ à 2 températures d'activation testées, de 50°C et 70°C pendant 20 minutes et à 3000 rpm.

L'évaluation n'est réalisée que 24 h après l'activation et 30 minutes après l'ajout dans la millbase du durcisseur dilué dans le xylène (voir tableau 4) et les peintures ainsi obtenues sont ajustées en viscosité d'application peinture, avec un mélange xylène/butanol (1/1 en poids) à environ 0,4 P ou environ 40 mPa.s (plus précisément à 0,37-0,38 P ou 37-38 mPa.s) mesuré sur le cône 4 à 25°C à 2500 s⁻¹ à l'aide d'un viscosimètre Brookfield® CAP 1000. Les proportions entre le durcisseur et le mélange de solvants sont définies dans le tableau 4 ci-dessous. La quantité de mélange xylène/butanol 1/1 utilisée pour l'ajustement de viscosité peut varier, mais en général de moins de 1% (de variation) d'un essai à l'autre.

Après l'ajustement, la peinture est mélangée/homogénéisée à 1500 rpm durant 2 minutes, puis laissée au repos pendant 30 minutes avant l'évaluation 24 h après.

**Tableau 3 : formulation « Millbase »**

| **A-COMPOSITION de la Millbase** | **Fonction** | **% poids** |
|---|---|---|
| Araldite® GZ 7071X75 | liant | 17,3 |
| Araldite® GY 783 BD | liant | 12,9 |
| BYK® A530 | Agent débullant | 0,5 |
| Disperbyk® 110 | Dispersant | 0,5 |
| Tiona® 595 (Titanium dioxyde) | Pigment | 1,9 |
| Bayferrox® 915 595 (oxyde de fer) | Pigment | 4,1 |
| ZP 10 (phosphate de zinc) | Pigment | 7,5 |
| Finntalc® MO5 | charge | 9,4 |
| Silice HPF6 | charge | 19,0 |
| n-Butanol | Solvant | 5,4 |
| Diamide 1, 2, 3 ou 4 | Additif de Rhéologie | 0,8 |
| **TOTAL** | | **79,3** |

**Tableau 4 : durcisseur**

| **B-Composition du durcisseur** | **% massique** |
|---|---|
| CRAYAMID® 140 | 8,8 |
| Xylène | 11,9 |
| **TOTAL** | **20,7** |

### 3) Evaluation de la rhéologie des formulations préparées des peintures époxy et résultats

Différentes formulations de peinture ont été réalisées avec les amides préparés 1 à 4 suivant les proportions présentées aux tableaux 3 et 4 et avec 2 températures d'activation examinées de 50 et 70°C suivant le protocole exposé plus haut.

Les résultats de résistance à la coulure et de rhéologie montrent que l'amide 3 à base de 1,3-bis(aminométhyl) cyclohexane a un effet thixotropique comparable ou au moins similaire à celui de l'amide 1 à base de m-xylylène diamine pour la même formulation activée à 50°C ou à 70°C.

Les résultats de résistance à la coulure et de rhéologie montrent que l'amide 4 à base de 1,3-bis(aminométhyl) cyclohexane a un effet thixotropique comparable ou au moins similaire à celui de l'amide 2 à base de m-xylylène diamine pour la même formulation activée à 50°C ou à 70°C.

En conséquence, les amides 3 et 4 à base de 1,3-bis(aminométhyl) cyclohexane présentent les caractéristiques nécessaires et très satisfaisantes d'un additif de rhéologie. La diamine 1,3-bis (aminométhyl) cyclohexane peut donc substituer la m-xylylène diamine dans le contexte de l'invention avec des résultats au moins aussi bons que ceux obtenus sans substitution mais, dans ce dernier cas, avec le problème de la toxicité dudit diamine non résolu et inconvénients cités.

Les résultats de résistance à la coulure sont présentés au tableau 5 et ceux de performances rhéologiques au tableau 6 ci-dessous.

**Tableau 5 : résultats de résistance à la coulure**

| **Température d'activation (°C)** | **Amide évalué** | **Résistance à la coulure (µm)** |
|---|---|---|
| **50** | 1 | 450 |
| **50** | 3 | 425 |
| **50** | 2 | 375 |
| **50** | 4 | 425 |
| **70** | 1 | 425 |
| **70** | 3 | 450 |
| **70** | 2 | 450 |
| **70** | 4 | 450 |

Les résultats d'évaluation rhéologique (viscosité et indice de thixotropie TI 1/10 et TI 5/50) sont présentés au tableau 6 ci-dessous.

**Tableau 6 : résultats rhéologiques**

| **Température d'activation (°C)** | **Amide évalué** | **Viscosité Brookfield à 25°C (mPa.s) à différentes vitesses de rotation allant de 1, 5, 10, 50 et 100 rpm** | | | | | **Indices de Thixotropie** | |
|---|---|---|---|---|---|---|---|---|
| | | **1** | **5** | **10** | **50** | **100** | **TI 1/10** | **TI 5/50** |
| 50 | 1 | 11600 | 3960 | 2640 | 1232 | 948 | 4,39 | 3,21 |
| 50 | 3 | 10200 | 3540 | 2360 | 1132 | 878 | 4,32 | 3,13 |
| 50 | 2 | 10200 | 3520 | 2380 | 1140 | 886 | 4,29 | 3,09 |
| 50 | 4 | 8800 | 3040 | 2060 | 1020 | 806 | 4,27 | 2,98 |
| 70 | 1 | 9600 | 3360 | 2260 | 1076 | 834 | 4,25 | 3,12 |
| 70 | 3 | 10400 | 3440 | 2280 | 1048 | 812 | 4,56 | 3,28 |
| 70 | 2 | 11800 | 3840 | 2540 | 1100 | 826 | 4,65 | 3,49 |
| 70 | 4 | 10000 | 3320 | 2200 | 1008 | 772 | 4,55 | 3,29 |

L'invention couvre les éléments suivants :
Elément 1. Diamide d'acide gras, caractérisé en ce qu'il comprend au moins un produit de réaction obtenu à partir d'un mélange réactionnel comprenant :
   a) au moins une diamine cycloaliphatique comprenant un cycle en 6 atomes de carbone et en particulier comprenant globalement, y compris ledit cycle, de 6 à 18 atomes de carbone, de préférence ladite diamine étant choisie parmi : la bis(aminométhyl)-1,3 cyclohexane (1,3-BAC), la bis(aminométhyl)-1,4 cyclohexane (1,4-BAC), la bis(aminométhyl)-1,2 cyclohexane (1,2-BAC) et leurs mélanges d'isomères, décahydronaphtalène diamines, isophorone diamines, plus préférentiellement la bis(aminométhyl)-1,3 cyclohexane (1,3-BAC), la bis(aminométhyl)-1,4 cyclohexane (1,4-BAC) et encore plus préférentiellement la la bis(aminométhyl)-1,3 cyclohexane (1,3-BAC),
   b) un hydroxy acide gras parmi l'acide 12-hydroxy stéarique (12-HSA), l'acide 9-hydroxy stéarique (9-HSA), l'acide 10-hydroxystéarique (10-HSA), l'acide 14-hydroxy eicosanéique (14-HEA) ou leurs mélanges binaires ou ternaires ou quaternaires, de préférence l'acide 12-hydroxy stéarique ou un mélange binaire ou ternaire de l'acide 12-hydroxy stéarique avec les autres hydroxyacides cités,
   c) au moins une deuxième diamine sélectionnée parmi les diamines primaires aliphatiques linéaires en C₂ à C₁₂, de préférence en C₂ à C₈, plus préférentiellement en C₂ à C₆,
   d) en option, au moins un monoacide sélectionné parmi les acides carboxyliques linéaires saturés et non hydroxylés en C₆ à C₁₈, de préférence de C₆ à C₁₅, plus préférentiellement de C₆ à C₁₂,
   e) en option, au moins une troisième diamine primaire différente de c) sélectionnée parmi les diamines aliphatiques linéaires en C₂ à C₁₂, de préférence en C₂ à C₈, plus préférentiellement en C₂ à C₆,
   f) au moins une diamine aromatique choisie parmi les xylylène diamines, de préférence parmi m-, p-xylylène diamines (m-, p-XDA), plus préférentiellement la m-xylylène diamine (m-XDA), ladite diamide aromatique pouvant être remplacée partiellement ou totalement par une diamine cycloaliphatique telle que définie selon a).
Elément 2. Diamide selon l'élément 1, caractérisée en ce que le % molaire de a/(a+f) est d'au moins 50% et va jusqu'à 100% et que le % molaire de (a+f)/(a+c+e+f) varie de 30 à 80% et de préférence de 30 à 65%.
Elément 3. Diamide d'acide gras selon l'élément 1 ou 2, caractérisé en ce que ladite diamine cycloaliphatique a), de préférence la 1,3-BAC ou 1,4-BAC, est en présence de xylylène diamines f), de préférence p-XDA ou m-XDA et plus préférentiellement m-XDA et que ladite diamine a) représente de 50 à 99% en moles du total en moles de diamines a) + xylylène diamine f).
Elément 4. Diamide d'acide gras selon l'élément 1 ou 2, caractérisé en ce que ladite diamine f) est absente, avec un rapport molaire a/(a+f) de 100%, de préférence avec ladite diamine cycloaliphatique a) étant la 1,4-BAC ou la 1,3-BAC.
Elément 5. Diamide selon l'un des éléments 1 à 4, caractérisé en ce qu'il comprend :
   i) un diamide consistant du produit de réaction de 1 mole de ladite diamine a) avec 2 moles dudit hydroxy acide b) comme défini selon l'élément 1, de préférence ledit hydroxy acide b) étant l'acide hydroxy-12 stéarique, et
   ii) un diamide consistant du produit de réaction de 1 mole de diamine c) avec 2 moles d'hydroxy acide b) comme défini selon l'élément 1, de préférence ledit hydroxy acide b) étant l'acide hydroxy-12 stéarique, et
   iii) un diamide consistant du produit de réaction de 1 mole de diamine selon e) et de 2 moles d'hydroxy acide b) comme défini selon l'élément 1, de préférence ledit hydroxy acide b) étant l'acide hydroxy-12 stéarique.
Elément 6. Diamide selon l'un des éléments 1 à 5, caractérisé en ce qu'il comprend au moins 2, de préférence au moins 3, produits de réaction différents tels qu'issus de la réaction entre lesdites diamines a), c), e) et éventuellement des xylylène diamines f) et les monoacides b) et éventuellement d).
Elément 7. Diamide selon l'un des éléments 1 à 6, caractérisé en ce que ledit monoacide d) est présent à un taux tel que le rapport molaire d/(b+d) est inférieur à 0,5 et de préférence il varie entre 0,02 et 0,5.
Elément 8. Diamide selon l'un des éléments 1 à 7, caractérisé en ce qu'il est sous forme de poudre micronisée, de préférence sous forme de poudre ayant une taille moyenne en volume inférieure à 20 µ, plus préférentiellement inférieure à 15 µ.
Elément 9. Utilisation du diamide tel que défini selon l'un des éléments 1 à 8 en tant qu'agent organogélateur en milieu solvant organique.
Elément 10. Utilisation selon l'élément 9, caractérisée en ce que ledit diamide est utilisé en tant qu'additif de rhéologie, en particulier sous forme de pâte préactivée préconcentrée dans un solvant organique.
Elément 11. Utilisation selon l'élément 9 ou 10, caractérisée en ce que ledit diamide est utilisé dans une composition de revêtements, en particulier dans les peintures, vernis, encres et enduits gélifiés (« gel coats ») ou dans une composition de colle ou d'adhésif ou d'agent décapant ou de moulage ou de cosmétique, plus particulièrement dans une composition de revêtements.
Elément 12. Utilisation selon l'élément 9 ou 10, caractérisée en ce que ledit diamide est utilisé dans une composition de mastic ou d'agent d'étanchéité.
Elément 13. Agent organogélateur, en particulier additif de rhéologie, caractérisé en ce qu'il comprend au moins un diamide tel que défini selon l'un des éléments 1 à 8.
Elément 14. Composition de liant organique, caractérisée en ce qu'elle comprend en tant qu'additif de rhéologie au moins un diamide tel que défini selon l'un des éléments 1 à 8.
Elément 15. Composition de liant organique selon l'élément 14, caractérisée en ce qu'il s'agit d'une composition de revêtements, en particulier de peintures, de vernis, d'encres et d'enduits gélifiés (« gel coats ») ou d'une composition de colle ou d'adhésif ou d'agent décapant ou de cosmétique.
Elément 16. Composition de liant organique selon l'élément 14, caractérisée en ce qu'il s'agit d'une composition de mastic ou d'agent d'étanchéité.
Elément 17. Composition de liant organique selon l'élément 14, caractérisée en ce qu'il s'agit d'une composition de moulage.
Elément 18. Composition selon l'élément 14, caractérisée en ce que ledit liant est sélectionné parmi : résines époxy, polyesters insaturés, vinyl esters, alkydes, résines silanées, polyuréthanes, polyesters-amides, résines acryliques solvantées, monomères et/ou oligomères acryliques ou élastomères chlorés ou élastomères non chlorés et polymères chlorés autres qu'élastomères chlorés.

## Revendications

1. Diamide d'acide gras, **caractérisé en ce qu'**il comprend les produits de réaction obtenus à partir d'un mélange réactionnel comprenant :
a) au moins une diamine cycloaliphatique comprenant un cycle en 6 atomes de carbone et en particulier comprenant globalement, y compris ledit cycle, de 6 à 18 atomes de carbone, de préférence ladite diamine étant choisie parmi : la bis(aminométhyl)-1,3 cyclohexane (1,3-BAC), la bis(aminométhyl)-1,4 cyclohexane (1,4-BAC), la bis(aminométhyl)-1,2 cyclohexane (1,2-BAC) et leurs mélanges d'isomères, décahydronaphtalène diamines, isophorone diamines, plus préférentiellement la bis(aminométhyl)-1,3 cyclohexane (1,3-BAC), la bis(aminométhyl)-1,4 cyclohexane (1,4-BAC), et encore plus préférentiellement la la bis(aminométhyl)-1,3 cyclohexane (1,3-BAC),
b) un hydroxy acide gras parmi l'acide 12-hydroxy stéarique (12-HSA), l'acide 9-hydroxy stéarique (9-HSA), l'acide 10-hydroxystéarique (10-HSA), l'acide 14-hydroxy eicosanéique (14-HEA) ou leurs mélanges binaires ou ternaires ou quaternaires, de préférence l'acide 12-hydroxy stéarique ou un mélange binaire ou ternaire de l'acide 12-hydroxy stéarique avec les autres hydroxyacides cités,
c) au moins une deuxième diamine sélectionnée parmi les diamines primaires aliphatiques linéaires en C₂ à C_{12,} de préférence en C₂ à C₈, plus préférentiellement en C₂ à C₆,
d) en option, au moins un monoacide sélectionné parmi les acides carboxyliques linéaires saturés et non hydroxylés en C₆ à C₁₈, de préférence de C₆ à C₁₅, plus préférentiellement de C₆ à C₁₂,
e) en option, au moins une troisième diamine primaire différente de c) sélectionnée parmi les diamines aliphatiques linéaires en C₂ à C₁₂, de préférence en C₂ à C₈, plus préférentiellement en C₂ à C₆,
f) au moins une diamine aromatique choisie parmi les xylylène diamines, de préférence parmi m-, p-xylylène diamines (m-, p-XDA), plus préférentiellement la m-xylylène diamine (m-XDA), ladite diamine aromatique étant remplacée totalement par une diamine cycloaliphatique telle que définie selon a), et
**en ce que** ladite diamine f) est absente, avec un rapport molaire a/(a+f) de 100%,
le % molaire de (a+f)/(a+c+e+f) varie de 30 à 80%.

2. Diamide selon la revendication 1, **caractérisée en ce que** le % molaire de (a+f)/(a+c+e+f) varie de 30 à 65%.

3. Diamide d'acide gras selon la revendication 1 ou 2, **caractérisé en ce que** ladite diamine cycloaliphatique a) est la 1,4-BAC ou la 1,3-BAC.

4. Diamide selon l'une des revendications 1 à 3, **caractérisé en ce qu'**il comprend :
i) un diamide consistant du produit de réaction de 1 mole de ladite diamine a) avec 2 moles dudit hydroxy acide b) comme défini selon la revendication 1, de préférence ledit hydroxy acide b) étant l'acide hydroxy-12 stéarique, et
ii) un diamide consistant du produit de réaction de 1 mole de diamine c) avec 2 moles d'hydroxy acide b) comme défini selon la revendication 1, de préférence ledit hydroxy acide b) étant l'acide hydroxy-12 stéarique, et
iii) un diamide consistant du produit de réaction de 1 mole de diamine selon e) et de 2 moles d'hydroxy acide b) comme défini selon la revendication 1, de préférence ledit hydroxy acide b) étant l'acide hydroxy-12 stéarique.

5. Diamide selon l'une des revendications 1 à 4, **caractérisé en ce qu'**il comprend au moins 2, de préférence au moins 3, produits de réaction différents tels qu'issus de la réaction entre lesdites diamines a), c) et e) et les monoacides b) et éventuellement d).

6. Diamide selon l'une des revendications 1 à 5, **caractérisé en ce que** ledit monoacide d) est présent à un taux tel que le rapport molaire d/(b+d) est inférieur à 0,5 et de préférence il varie entre 0,02 et 0,5.

7. Diamide selon l'une des revendications 1 à 6, **caractérisé en ce qu'**il est sous forme de poudre micronisée, de préférence sous forme de poudre ayant une taille moyenne en volume inférieure à 20 µm, plus préférentiellement inférieure à 15 µm.

8. Utilisation du diamide tel que défini selon l'une des revendications 1 à 7 en tant qu'agent organogélateur en milieu solvant organique.

9. Utilisation selon la revendication 8, **caractérisée en ce que** ledit diamide est utilisé en tant qu'additif de rhéologie, en particulier sous forme de pâte préactivée préconcentrée dans un solvant organique.

10. Utilisation selon la revendication 8 ou 9, **caractérisée en ce que** ledit diamide est utilisé dans une composition de revêtements, en particulier dans les peintures, vernis, encres et enduits gélifiés (« gel coats ») ou dans une composition de colle ou d'adhésif ou d'agent décapant ou de moulage ou de cosmétique, plus particulièrement dans une composition de revêtements.

11. Utilisation selon la revendication 8 ou 9, **caractérisée en ce que** ledit diamide est utilisé dans une composition de mastic ou d'agent d'étanchéité.

12. Agent organogélateur, en particulier additif de rhéologie, **caractérisé en ce qu'**il comprend au moins un diamide tel que défini selon l'une des revendications 1 à 7.

13. Composition de liant organique, **caractérisée en ce qu'**elle comprend en tant qu'additif de rhéologie au moins un diamide tel que défini selon l'une des revendications 1 à 7.

14. Composition de liant organique selon la revendication 13, **caractérisée en ce qu'**il s'agit d'une composition de revêtements, en particulier de peintures, de vernis, d'encres et d'enduits gélifiés (« gel coats ») ou d'une composition de colle ou d'adhésif ou d'agent décapant ou de cosmétique.

15. Composition selon la revendication 14, **caractérisée en ce que** ledit liant est sélectionné parmi : résines époxy, polyesters insaturés, vinyl esters, alkydes, résines silanées, polyuréthanes, polyesters-amides, résines acryliques solvantées, monomères et/ou oligomères acryliques ou élastomères chlorés ou élastomères non chlorés et polymères chlorés autres qu'élastomères chlorés.

## Patentansprüche

1. Fettsäurediamid, **dadurch gekennzeichnet, dass** es die Reaktionsprodukte umfasst, die aus einer Reaktionsmischung erhalten werden, die Folgendes umfasst:
a) mindestens ein cycloaliphatisches Diamin mit einem Ring aus 6 Kohlenstoffatomen und insbesondere insgesamt, einschließlich des Rings, 6 bis 18 Kohlenstoffatomen, wobei das Diamin vorzugsweise aus 1,3-Bis(aminomethyl)-cyclohexan (1,3-BAC), 1,4-Bis(aminomethyl)-cyclohexan (1,4-BAC), 1,2-Bis(aminomethyl)-cyclohexan (1,2-BAC) und Isomerengemischen davon, Decahydronaphthalindiaminen, Isophorondiaminen, weiter bevorzugt 1,3-Bis(aminomethyl)cyclohexan (1,3-BAC), 1,4-Bis(aminomethyl)cyclohexan (1,4-BAC) und noch weiter bevorzugt 1,3-Bis(aminomethyl)cyclohexan (1,3-BAC), ausgewählt ist,
b) eine Hydroxyfettsäure aus 12-Hydroxystearinsäure (12-HSA), 9-Hydroxystearinsäure (9-HSA), 10-Hydroxystearinsäure (10-HSA), 14-Hydroxyeicosansäure (14-HEA) oder binären oder ternären oder quaternären Gemischen davon, vorzugsweise 12-Hydroxystearinsäure oder einem binären oder ternären Gemisch von 12-Hydroxystearinsäure mit den anderen genannten Hydroxysäuren,
c) mindestens ein zweites Diamin, das aus linearen aliphatischen primären C₂- bis C₁₂-, vorzugsweise C₂- bis C₈- und weiter bevorzugt C₂- bis C₆-Diaminen ausgewählt ist,
d) gegebenenfalls mindestens eine Monosäure, die aus gesättigten und nicht hydroxylierten linearen C₆- bis C₁₈-, vorzugsweise C₆- bis C₁₅- und weiter bevorzugt C₆- bis C₁₂-Carbonsäuren ausgewählt ist,
e) gegebenenfalls mindestens ein drittes primäres Diamin, das von c) verschieden ist und aus linearen aliphatischen C₂- bis C₁₂-, vorzugsweise C₂- bis C₈- und weiter bevorzugt C₂- bis C₆-Diaminen ausgewählt ist,
f) mindestens ein aromatisches Diamin, das aus Xylylendiaminen, vorzugsweise aus m-, p-Xylylendiamin (m-, p-XDA), weiter bevorzugt m-Xylylendiamin (m-XDA), ausgewählt ist, wobei das aromatische Diamin vollständig durch ein cycloaliphatisches Diamin gemäß a) ersetzt ist, und
dadurch, dass
- das Diamin f) fehlt, wobei das Molverhältnis a/(a+f) 100 % beträgt,
- der Molprozentanteil von (a+f)/(a+c+e+f) im Bereich von 30 bis 80 % liegt.

2. Diamid nach Anspruch 1, **dadurch gekennzeichnet, dass** der Molprozentanteil von (a+f)/(a+c+e+f) im Bereich von 30 bis 65 % liegt.

3. Fettsäurediamid nach Anspruch 1 oder 2, **dadurch gekennzeichnet, dass** es sich bei dem cycloaliphatischen Diamin a) um 1,4-BAC oder 1,3-BAC handelt.

4. Diamid nach einem der Ansprüche 1 bis 3, **dadurch gekennzeichnet, dass** es Folgendes umfasst:
i) ein Diamid, das aus dem Reaktionsprodukt von 1 mol des Diamins a) mit 2 mol der Hydroxysäure b) gemäß Anspruch 1 besteht, wobei es sich bei der Hydroxysäure b) vorzugsweise um 12-Hydroxystearinsäure handelt, und
ii) ein Diamid, das aus dem Reaktionsprodukt von 1 mol Diamin c) mit 2 mol Hydroxysäure b) gemäß Anspruch 1 besteht, wobei es sich bei der Hydroxysäure b) vorzugsweise um 12-Hydroxystearinsäure handelt, und
iii) ein Diamid, das aus dem Reaktionsprodukt von 1 mol Diamin gemäß e) und 2 mol Hydroxysäure b) gemäß Anspruch 1 besteht, wobei es sich bei der Hydroxysäure b) vorzugsweise um 12-Hydroxystearinsäure handelt.

5. Diamid nach einem der Ansprüche 1 bis 4, **dadurch gekennzeichnet, dass** es mindestens 2, vorzugsweise mindestens 3, verschiedene Reaktionsprodukte umfasst, wie sie aus der Reaktion zwischen den Diaminen a), c) und e) und den Monosäuren b) und gegebenenfalls d) stammen.

6. Diamid nach einem der Ansprüche 1 bis 5, **dadurch gekennzeichnet, dass** die Monosäure d) in einem solchen Gehalt vorliegt, dass das Molverhältnis d/ (b+d) kleiner als 0,5 ist und vorzugsweise im Bereich zwischen 0,02 und 0,5 liegt.

7. Diamid nach einem der Ansprüche 1 bis 6, **dadurch gekennzeichnet, dass** es in Form eines mikronisierten Pulvers vorliegt, vorzugsweise in Form eines Pulvers mit einer volumenmittleren Größe von weniger als 20 pm, weiter bevorzugt weniger als 15 µm.

8. Verwendung des Diamids gemäß einem der Ansprüche 1 bis 7 als organischer Gelbildner in einem organischen Lösungsmittelmedium.

9. Verwendung nach Anspruch 8, **dadurch gekennzeichnet, dass** das Diamid als Rheologieadditiv verwendet wird, insbesondere in Form einer in einem organischen Lösungsmittel vorkonzentrierten voraktivierten Paste.

10. Verwendung nach Anspruch 8 oder 9, **dadurch gekennzeichnet, dass** das Diamid in einer Beschichtungszusammensetzung, insbesondere in Anstrichmitteln, Klarlacken, Tinten und Gelcoats, oder in einer Leim- oder Klebstoff- oder Beizmittelzusammensetzung oder Formmasse oder Kosmetikzusammensetzung, insbesondere in einer Beschichtungszusammensetzung, verwendet wird.

11. Verwendung nach Anspruch 8 oder 9, **dadurch gekennzeichnet, dass** das Diamid in einer Kittzusammensetzung oder einem Dichtungsmittel verwendet wird.

12. Organischer Gelbildner, insbesondere Rheologieadditiv, **dadurch gekennzeichnet, dass** er mindestens ein Diamid gemäß einem der Ansprüche 1 bis 7 umfasst.

13. Organische Bindemittelzusammensetzung, **dadurch gekennzeichnet, dass** sie als Rheologieadditiv mindestens ein Diamid gemäß einem der Ansprüche 1 bis 7 umfasst.

14. Organische Bindemittelzusammensetzung nach Anspruch 13, **dadurch gekennzeichnet, dass** es sich um eine Beschichtungszusammensetzung, insbesondere eine Anstrichmittel-, Klarlack-, Tinten- oder Gelcoat-Zusammensetzung, oder eine Leim- oder Klebstoff- oder Beizmittel- oder Kosmetikzusammensetzung handelt.

15. Zusammensetzung nach Anspruch 14, **dadurch gekennzeichnet, dass** das Bindemittel aus Epoxidharzen, ungesättigten Polyestern, Vinylestern, Alkyden, Silanharzen, Polyurethanen, Polyesteramiden, lösungsmittelhaltigen Acrylharzen, Acrylmonomeren und/oder -oligomeren oder chlorierten Elastomeren oder nichtchlorierten Elastomeren und chlorierten Polymeren, die von chlorierten Elastomeren verschieden sind, ausgewählt ist.

## Claims

1. Fatty acid diamide, **characterized in that** it comprises reaction products derived from a reaction mixture comprising:
a) at least one cycloaliphatic diamine comprising a ring of 6 carbon atoms and in particular globally comprising, including the said ring, from 6 to 18 carbon atoms, the said diamine preferably being chosen from: 1,3-bis(aminomethyl)cyclohexane (1,3-BAC), 1,4-bis(aminomethyl)cyclohexane (1,4-BAC), 1,2-bis(aminomethyl)cyclohexane (1,2-BAC) and isomer mixtures thereof, decahydronaphthalenediamines, isophoronediamines, more preferentially 1,3-bis(aminomethyl)cyclohexane (1,3-BAC), 1,4-bis(aminomethyl)cyclohexane (1,4-BAC), and even more preferentially 1,3-bis(aminomethyl)-cyclohexane (1,3-BAC),
b) a fatty hydroxy acid from 12-hydroxystearic acid (12-HSA), 9-hydroxystearic acid (9-HSA), 10-hydroxystearic acid (10-HSA), 14-hydroxy-eicosanoic acid (14-HEA) or binary or ternary or quaternary mixtures thereof, preferably 12-hydroxystearic acid or a binary or ternary mixture of 12-hydroxystearic acid with the other hydroxy acids mentioned,
c) at least a second diamine selected from linear primary aliphatic C₂ to C₁₂, preferably C₂ to C₈ and more preferentially C₂ to C₆ diamines,
d) optionally, at least one monoacid selected from saturated and non-hydroxylated linear C₆ to C₁₈, preferably C₆ to C₁₅ and more preferentially C₆ to C₁₂ carboxylic acids,
e) optionally, at least a third primary diamine different from c) selected from linear aliphatic C₂ to C₁₂, preferably C₂ to C₈ and more preferentially C₂ to C₆ diamines,
f) at least one aromatic diamine chosen from xylylenediamines, preferably from m-, p-xylylenediamines (m-, p-XDA), more preferentially m-xylylenediamine (m-XDA), the said aromatic diamine being completely replaced with a cycloaliphatic diamine as defined in point a) and **in that** the said diamine f) is absent, with an a/(a+f) molar ratio of 100%, the molar % of (a+f)/(a+c+e+f) ranges from 30% to 80%.

2. Diamide according to Claim 1, **characterized in that** the molar % of (a+f)/(a+c+e+f) ranges from 30% to 65%.

3. Fatty acid diamide according to Claim 1 or 2, **characterized in that** the said cycloaliphatic diamine a) is 1,4-BAC or 1,3-BAC.

4. Diamide according to one of Claims 1 to 3, **characterized in that** it comprises:
i) a diamide consisting of the reaction product of 1 mol of the said diamine a) with 2 mol of the said hydroxy acid b) as defined according to Claim 1, the said hydroxy acid b) preferably being 12-hydroxystearic acid, and
ii) a diamide consisting of the reaction product of 1 mol of diamine c) with 2 mol of hydroxy acid b) as defined according to Claim 1, the said hydroxy acid b) preferably being 12-hydroxystearic acid, and
iii) a diamide consisting of the reaction product of 1 mol of diamine according to e) and 2 mol of hydroxy acid b) as defined according to Claim 1, the said hydroxy acid b) preferably being 12-hydroxystearic acid.

5. Diamide according to one of Claims 1 to 4, **characterized in that** it comprises at least 2, preferably at least 3, different reaction products as resulting from the reaction between the said diamines a), c) and e) and the monoacids b) and optionally d).

6. Diamide according to one of Claims 1 to 5, **characterized in that** the said monoacid d) is present in a proportion such that the molar ratio d/(b+d) is less than 0.5 and preferably ranges from between 0.02 and 0.5.

7. Diamide according to one of Claims 1 to 6, **characterized in that** it is in the form of a micronized powder, preferably in the form of a powder with a mean size by volume of less than 20 microns, more preferentially less than 15 microns.

8. Use of the diamide as defined according to one of Claims 1 to 7 as an organogelling agent in an organic solvent medium.

9. Use according to Claim 8, **characterized in that** the said diamide is used as a rheology additive, in particular in the form of a preactivated paste pre-concentrated in an organic solvent.

10. Use according to Claim 8 or 9, **characterized in that** said diamide is used in a coating composition, in particular in paints, varnishes, inks and gel coats or in a glue or adhesive or stripping agent or moulding or cosmetic composition, more particularly in a coating composition.

11. Use according to Claim 8 or 9, **characterized in that** the said diamide is used in a mastic composition or sealing agent.

12. Organogelling agent, in particular rheology additive, **characterized in that** it comprises at least one diamide as defined according to one of Claims 1 to 7 .

13. Organic binder composition, **characterized in that** it comprises as rheology additive at least one diamide as defined according to one of Claims 1 to 7.

14. Organic binder composition according to Claim 13, **characterized in that** it is a coating composition, in particular a paint, varnish, ink or gel coat composition or a glue or adhesive or stripping agent or cosmetic composition.

15. Composition according to Claim 14, **characterized in that** the said binder is selected from: epoxy resins, unsaturated polyesters, vinyl esters, alkyds, silane resins, polyurethanes, polyester amides, solvent-based acrylic resins, acrylic monomers and/or oligomers or chlorinated elastomers or non-chlorinated elastomers and chlorinated polymers other than chlorinated elastomers.
